# DEMANDE DE BREVET EUROPEEN

(11) **EP 1 317 926 A1**
(43) Date de publication de la demande: **11.06.2003**
(21) Numéro de dépôt: 03290499.7
(22) Date de dépôt: 19.07.2001
(51) Int. Cl.: A61K 33/00, A61K 9/00, A61K 9/12

(54) **Xénon inhalé pour traiter la douleur**

(30) Priorité: 03.08.2000 FR 0010065
(62) Demande divisionnaire de: 01401947.5
(71) Demandeur: Air Liquide Sante (International), 75341 Paris Cedex 07 (FR)
(72) Inventeur: Lecourt, Laurent, 92310 Sevres (FR); Lemaire, Marc, 75014 Paris (FR); Lescure, Franck, 75013 Paris (FR)
(74) Mandataire: Pittis, Olivier

(57) **Abrégé**

Utilisation d'au moins un produit actif choisi parmi le paracétamol, l'acide acétylsalicylique, l'acide aryl carboxylique, les corticostéroïdes, les minéralostéroïdes, les anti-inflammatoires non stéroïdiens et leurs dérivés, la codéine et ses dérivés, la morphine et les morphinomimétiques pour fabriquer un médicament inhalable destiné, en association avec au moins du xénon gazeux, à traiter ou à prévenir la douleur chez l'homme ou l'animal. Médicament inhalable contenant du xénon et un produit actif destiné à traiter ou à prévenir la douleur, en particulier un antalgique, un composé à action anti-inflammatoire ou un anti-pyrétique.

## Description

L'invention porte sur l'utilisation de xénon gazeux ou d'un mélange gazeux contenant du xénon, d'une part, et d'un produit ou substance thérapeutiquement actif, d'autre part, pour fabriquer tout ou partie d'un médicament inhalable, en particulier un aérosol, destiné à traiter ou à prévenir la douleur.

Actuellement, pour lutter contre la douleur, on administre le médicament ou la substance médicamenteuse anti-douleur soit par voie entérale, soit par voie parentérale de manière à ce que celle-ci puisse aller agir et atténuer complètement ou partiellement la sensation de douleur.

La voie entérale consiste à administrer un produit ou une substance active par les voies digestives du patient, c'est-à-dire soit de lui faire absorber ou avaler par la bouche le médicament par exemple sous forme d'une poudre, d'une pilule, d'un comprimé, d'un liquide ; soit d'introduire la substance active par l'anus, par exemple sous forme de suppositoire.

Toutefois, la voie entérale est plus adaptée au traitement de la douleur chronique qu'à la prise en charge de la douleur aiguë .

Parmi les voies d'administration des médicaments visant à lutter contre la douleur, notamment la douleur aiguë, la voie parentérale est la plus utilisée .

Administrer un médicament ou une substance thérapeutiquement active à un malade par la voie parentérale consiste habituellement à injecter ledit médicament audit patient au moyen d'une seringue munie d'une aiguille.

Une injection par voie parentérale peut être notamment intradermique, intravasculaire, intramusculaire, sous-cutanée......

Cependant, les problèmes et risques engendrés par une administration de médicament par voie parentérale sont de plusieurs types, à savoir les risques pour les patients, les risques pour le personnel soignant et le rapport efficacité / tolérance des produits administrés.

Plus précisément, en ce qui les risques ou inconforts pour les patients, on comprend aisément que l'effraction cutanée causée par le système d'injection, en particulier l'aiguille, est souvent vécue comme une agression, en particulier chez les enfants, mais aussi chez les patients dont le réseau veineux est d'accès difficile, tels les sujets âgés, les patients atteints de pathologies lourdes (cancer, immunodéficience acquise ou congénitale...), toxicomanes... Ce sentiment est lié à la douleur répétée, créée par l'effraction cutanée lors de l'injection.

En dehors de cette sensation d'agression, l'acte en lui même est potentiellement dangereux. En effet, le risque d'effraction veineuse, c'est-à-dire de détérioration des vaisseaux sanguins, est non négligeable lors d'une injection de type intra-musculaire ou intra-dermique.

Un autre risque potentiel est celui de l'hémorragie qui peut en résulter et la formation d'un hématome sous cutané, en particulier chez les patients hémophiles ou soumis à un traitement par des produits anti-coagulants .

En outre, certains patients sont dits "impiquables" du fait de l'état de leur réseau veineux ; du fait de l'âge, c'est le plus souvent le cas des enfants ou des personnes âgées ; de l'abus de drogues par exemple les toxicomanes ; de multiples ponctions ou injections dans le cadre de traitements lourds, tels les patients traités pour des cancers ou des hémopathies malignes, les patients obèses...

Pour toutes ces populations de malades, le risque est celui d'une lymphangite qui peut se sur-infecter chez les patients immunodéprimés, notamment ceux atteints d'une pathologie du type HIV, cancer, hémopathies... Chez ces patients, le risque majeur encouru est constitué par l'infection sur cathéter, pouvant entraîner une septicémie avec des dégâts organiques au niveau du coeur, foie, rein, poumon..., nécessitant l'ablation du cathéter, la mise en place d'une antibiothérapie lourde et non dénuée de risque toxique et surtout retardant la poursuite du traitement de la maladie initiale.

Il va de soi que tous ces risques se multiplient et croissent avec la chronicité des maladies comme dans les cancers, les maladies inflammatoires chroniques...

Par ailleurs, il existe aussi des risques évidents pour le personnel médical soignant (infirmières, sages-femmes, médecins), qui doivent absolument éviter tout contact avec le sang contaminé du malade lors de l'injection du médicament.

En outre, il a été démontré qu'un médicament administré par voie entérale ou parentérale est métabolisé dans la majorité des cas, c'est ce que l'on appelle l'effet de premier passage hépatique.

Par tenir compte de cet effet, il est classiquement nécessaire soit d'augmenter la dose initiale de principe actif tout en sachant que seule une faible partie dudit principe actif sera réellement efficace car non métabolisé, soit d'utiliser une pro-drogue dont le métabolisme donnera naissance à la drogue active.

Toutefois, dans tous les cas, le rapport de la dose active à la dose administrée est faible, ce qui par ailleurs augmente les risques toxiques des médicaments en diminuant le rapport bénéfices / risques.

En d'autres termes, le problème qui se pose est de proposer une composition thérapeutiquement efficace pour lutter contre la douleur qui puisse être administrée de façon sûre tant pour le patient que pour le personnel soignant, et qui ne présente donc pas les problèmes et risques susmentionnés.

De plus, ladite composition thérapeutiquement efficace ou médicament doit être aussi facile à produire au plan industriel ou pharmaceutique.

La solution à ces problèmes apportée par les inventeurs de la présente invention repose essentiellement sur l'utilisation de la voie inhalée pour administrer les substances actives au malade et ce, à la place des voies entérale et parentérale classiquement utilisées.

La voie inhalée ou aérosolthérapie peut permettre, dans certaines conditions, de résoudre les problèmes liés à l'usage de la voie parentérale et, dans une moindre mesure, de la voie entérale.

En effet, un intérêt principal de la voie inhalée est de permettre le passage de la drogue ou substance thérapeutiquement active directement dans le sang artériel du malade, via une surface de passage et d'échange importante, en particulier la membrane alvéolo-capillaire du tractus respiratoire, et donc en évitant le métabolisme hépatique ou rénal qui engendre habituellement une dégradation d'une partie du médicament et nécessite alors d'augmenter les doses administrées pour prendre en compte ces pertes de substance active.

Administrer le médicament par voie inhalée, sous forme d'aérosol, permet non seulement de minimiser les risques qui existent avec les administrations par voie parentérale et ce, sans perdre l'efficacité du médicament, mais aussi de traiter certaines pathologies qui ne peuvent pas l'être par voie entérale alors que les malades atteints de ces pathologies font partie de populations pour lesquelles la voie parentérale n'est pas ou mal adaptée ou utilisable.

Jusqu'à aujourd'hui, la voie inhalée était essentiellement utilisée pour traiter des pathologies ou affections locales du tractus respiratoire, à savoir les maladies pulmonaires et bronchiales, tel l'asthme.

A ce titre, on peut citer les documents EP-A-680315, EP-A-655237, WO-A-98/7419, EP-A-550031, EP-A-384371, EP-A-658101, EP-A-625046, EP-A-616525, EP-A-556256, EP-A-556239, EP-A-616523, EP-A-539674, EP-A-789557, EP-A-799024, EP-A-741588, WO-A-99/53901 et WO-A-99/55319.

Or, l'utilisation d'aérosols médicamenteux dans le traitement de la douleur n'a, jusqu'à maintenant, jamais été décrit ou suggéré, en particulier pour les pathologies non-pulmonaires et non-bronchiales.

La solution de l'invention repose sur une utilisation d'au moins un produit actif choisi parmi le paracétamol, l'acide acétylsalicylique, l'acide aryl carboxylique, les corticostéroïdes, les minéralostéroïdes, les anti-inflammatoires non stéroïdiens et leurs dérivés, la codéine et ses dérivés, la morphine et les morphinomimétiques pour fabriquer un médicament inhalable destiné, en association avec au moins du xénon gazeux, à traiter ou à prévenir la douleur chez l'homme ou l'animal.

Selon le cas, l'utilisation de l'invention peut comprendre l'une ou plusieurs des caractéristiques suivantes :
- le produit actif est choisi parmi les antalgiques.
- le produit actif est choisi parmi les composés à action anti-inflammatoire.
- le produit actif est choisi parmi les anti-pyrétiques.
- le médicament inhalable est sous forme d'un aérosol comprenant ledit gaz et ledit produit actif sous forme de poudre, de liquide ou d'un mélange poudre/liquide.
- le médicament inhalable contient une quantité thérapeutiquement efficace de produit actif et dans laquelle l'association dudit au moins un gaz avec ledit au moins un produit actif conduit à un effet synergique.
- le médicament inhalable contient, en outre, au moins un gaz choisi parmi l'hélium, l'oxygène, l'azote, l'hydrogène, le monoxyde de carbone (CO), le dioxyde de carbone (CO₂), l'argon, le krypton, le monoxyde d'azote (NO), le protoxyde d'azote (N₂O) et les mélanges de plusieurs de ces gaz
- le médicament inhalable contient, en outre, au moins un gaz choisi parmi le CO et le NO, de préférence un mélange gazeux contenant NO et CO, et au moins un produit actif à action anti-inflammatoire .
- le médicament inhalable contient au moins un gaz choisi parmi le O₂ et le N₂O, de préférence un mélange gazeux contenant O₂ et N₂O, et au moins un produit actif antalgique ou morphinique.

Dans le cadre de la présente invention, les termes "produit actif' sont utilisés dans un sens général pour désigner tout composé, toute molécule, tout principe actif, toute substance, toute composition, organique, minérale ou végétale, ou leurs mélanges, sous quelque forme que ce soit, à savoir liquide, solide, mélange liquide/solide, suspension, dilution, émulsion ayant une activité thérapeutique non nulle dans le traitement de la douleur et dont l'administration au patient par voie inhalée, via les voies aériennes du patient, va engendrer une diminution de sa perception ou de sa sensation de douleur.

Toutefois, les pathologies broncho-pulmonaires, tel l'asthme, sont exclues du champ de protection de la présente invention.

De façon plus générale, le médicament inhalable de l'invention est formé d'une association d'au moins un gaz et d'au moins un produit actif à action analgésique, ledit produit actif étant choisi parmi le paracétamol, l'acide acétylsalicylique, l'acide arylcarboxylique, les corticostéroïdes, les minéralostéroïdes, les anti-inflammatoires non stéroïdiens et leurs dérivés, la codéine et ses dérivés, la morphine et les morphinomimétiques.

Dans le cadre de l'invention, le gaz est le xénon mais le médicament peut aussi comprendre un gaz choisi parmi l'hélium, l'oxygène, l'azote, l'hydrogène, le dioxyde de carbone, l'argon, le krypton, le monoxyde de carbone (CO), le monoxyde d'azote (NO), le protoxyde d'azote (N₂O), et les mélanges de plusieurs de ces gaz.

Utiliser la voie inhalée pour administrer un médicament anti-douleur présente aussi d'autres avantages pour le patient, à savoir qu'en évitant un acte douloureux, la voie inhalée permet de réduire le risque infectieux, inflammatoire et hémorragique.

De plus, la voie inhalée permet de réduire la dose administrée de principe actif, permet d'associer des formes galéniques différentes de principes actifs synergiques et permet de vérifier l'observance du traitement par le malade, notamment si l'on utilise un appareil d'aérosolthérapie adapté, tel l'appareil commercialisé par AIR LIQUIDE SANTE sous la dénomination commerciale OPTINEB™. Toutefois, d'autres dispositifs peuvent être utilisés comme expliqué ci-après.

Par ailleurs, grâce à l'administration du médicament par la voie inhalée, il est désormais possible ou plus facile de soigner les populations de malades dit "à risques" ou "difficiles", tels les patients immunodéprimés, c'est-à-dire atteints de cancers, d'hémopathies ou d'hépatites B ou C, les toxicomanes, les malades atteints du virus HIV, les patients hémophiles ou sous anti-coagulants, ou encore les patients dont le réseau veineux est difficile d'accès, tels les enfants, les personnes âgées ou obèses.

Dans le cadre de l'invention, le médicament peut être préparé sous une forme stable et être conditionné, par exemple, dans des récipients sous pression, tels des distributeurs de produits aérosols à actionnement par pression digitale, comme décrit notamment dans le document EP-A-708805.

Selon le cas, le médicament peut aussi être préparé juste avant son administration au patient, c'est-à-dire extemporanément.

De façon générale, lors de la mise en oeuvre d'un traitement par aérosolthérapie selon l'invention, les gaz utilisables peuvent être utilisés de plusieurs manières, qui peuvent se combiner entre elles, à savoir :
- pour réaliser la nébulisation si l'on considère un système de nébulisation pneumatique,
- en tant que vecteur du nébulisât et ce, quelque soit le système de nébulisation employé,
- pour les propriétés thérapeutiques de ces gaz sur la cible thérapeutique elle-même ou sur le principe actif nébulisé, en particulier lorsque le produit actif et le gaz agissent de façon synergique.

Toutefois, nébulisation et vectorisation du gaz sont parfois confondues, c'est-à-dire qu'un même gaz nébulise le produit actif et le vectorise ensuite dans le tractus pulmonaire du patient.

A l'inverse, lorsqu'il est souhaitable ou nécessaire de bien distinguer entre ces deux fonctions, on peut utiliser par exemple un système tel le dispositif OPTI+™ commercialisé par la Société Vitalaire qui peut être couplé, d'une part, à un ventilateur médical pour délivrer une pression positive d'un gaz respiratoire et, d'autre part, à un système de nébulisation à l'oxygène, par exemple.

On estime qu'une nébulisation efficace pour atteindre les alvéoles des poumons doit générer des particules d'aérosol d'environ 1 à 5 µm.

La nature physique du gaz est donc à prendre en compte et on peut estimer, hors considérations physiologiques, que tout gaz de nature physique proche de l'oxygène sera un bon gaz nébuliseur. L'oxygène ou des mélanges gazeux à base d'oxygène sont envisageables car ils permettent d'utiliser un même gaz pour la vectorisation et la nébulisation

De la même façon, certains gaz modifiant les paramètres hémodynamiques cardio-pulmonaire (débit cardiaque, volémie...) sont susceptibles d'influer sur l'efficacité de la nébulisation.

De façon analogue, certains gaz ayant des effets thérapeutiques peuvent être utilisés comme vecteurs du nébulisât de produit actif contenant le ou les principes actifs et ce, de manière à augmenter l'effet desdits principes actifs nébulisés grâce à une action synergique du gaz et desdits principes actifs.

Ainsi, les associations données dans le tableau suivant peuvent être envisagées dans le cadre du traitement de la douleur.

**Tableau :**

| Associations à effet synergique potentiel | |
|---|---|
| **GAZ** | **PRINCIPES ACTIFS** |
| | Anti-Inflammatoire Non Stéroïdien |
| Xénon | Anti-Inflammatoire Stéroïdien |
| | Aspirine |
| | Morphine |
| | Morphinomimétiques |
| | Codéïne et dérivés |
| | Paracétamol |

Le principe actif peut être potentialisé par le gaz de nébulisation. Par exemple, un principe actif stocké sous une forme réduite plus stable peut être potentialisé au moyen d'une nébulisation par un gaz plus ou moins oxydant, tel O₂, NO, CO ou CO₂.

Cette approche présente au moins les avantages d'une meilleure stabilité du principe actif libéré dans l'organisme et d'un ciblage plus efficace des cellules visées par le traitement.

Cette approche est valable pour n'importe quel traitement de la douleur que l'on veut faire agir de façon ciblée.

## Revendications

1. Utilisation d'au moins un produit actif choisi parmi le paracétamol, l'acide acétylsalicylique, l'acide aryl carboxylique, les corticostéroïdes, les minéralostéroïdes, les anti-inflammatoires non stéroïdiens et leurs dérivés, la codéine et ses dérivés, la morphine et les morphinomimétiques pour fabriquer un médicament inhalable destiné, en association avec au moins du xénon gazeux, à traiter ou à prévenir la douleur chez l'homme ou l'animal.

2. Utilisation selon la revendication 1, dans laquelle le produit actif est choisi parmi les antalgiques.

3. Utilisation selon la revendication 1, dans laquelle le produit actif est choisi parmi les composés à action anti-inflammatoire.

4. Utilisation selon la revendication 1, dans laquelle le produit actif est choisi parmi les anti-pyrétiques.

5. Utilisation selon l'une des revendications 1 à 4, dans laquelle le médicament inhalable est sous forme d'un aérosol comprenant ledit gaz et ledit produit actif sous forme de poudre, de liquide ou d'un mélange poudre/liquide.

6. Utilisation selon l'une des revendications 1 à 5, dans laquelle le médicament inhalable contient une quantité thérapeutiquement efficace de produit actif.

7. Utilisation selon l'une des revendications 1 à 6, dans laquelle l'association dudit au moins un gaz avec ledit au moins un produit actif conduit à un effet synergique.

8. Utilisation selon l'une des revendications 1 à 7, dans laquelle le médicament inhalable contient au moins un gaz choisi parmi le CO et le NO, de préférence un mélange gazeux contenant NO et CO, et au moins un produit actif à action anti-inflammatoire .

9. Utilisation selon l'une des revendications 1 à 8, dans laquelle le médicament inhalable contient, en outre, au moins un gaz choisi parmi le O₂ et le N₂O,

10. Utilisation selon l'une des revendications 1 à 9, dans laquelle le médicament inhalable contient, en outre, O₂ et N₂O, et au moins un produit actif antalgique ou morphinique.

11. Médicament inhalable formé d'une association d'au moins du xénon gazeux et d'au moins un produit actif à action analgésique choisi parmi le paracétamol, l'acide acétylsalicylique, l'acide arylcarboxylique, les corticostéroïdes, les minéralostéroïdes, les anti-inflammatoires non stéroïdiens et leurs dérivés, la codéine et ses dérivés, la morphine et les morphinomimétiques.

12. Médicament selon la revendication 11, **caractérisé en ce qu'**il contient une quantité thérapeutiquement efficace de produit actif.

13. Médicament selon la revendication 11, **caractérisé en ce qu'**il contient, en outre, de l'hélium, de l'oxygène, de l'azote, de l'hydrogène, du monoxyde de carbone (CO), du dioxyde de carbone (CO₂), de l'argon, du krypton, du monoxyde d'azote (NO) ou du protoxyde d'azote (N₂O).
